# EUROPEAN PATENT SPECIFICATION

(11) **EP 2 217 317 B1**
(45) Date of publication and mention of the grant of the patent: **04.03.2015**
(21) Application number: 08846367.4
(22) Date of filing: 06.11.2008
(51) Int. Cl.: A61M 37/00

(54) **HYPERBARIC WOUND TREATMENT DEVICE**
HYPERBARE WUNDBEHANDLUNGSVORRICHTUNG
DISPOSITIF DE TRAITEMENT DE BLESSURE HYPERBARE

(30) Priority: 06.11.2007 US 2085 P
(43) Date of publication of application: 18.08.2010
(73) Proprietor: AOTI Limited, Galway (IE)
(72) Inventor: LOORI, Phillip, Farmingdale NJ 07727 (US); MISZENCIN, Steve, Hulmeville PA 19047 (US)
(74) Representative: Clark, Jane Anne
(86) International application number: PCT/US2008/012625
(87) International publication number: WO 2009/061503

(56) References cited:
- WO-A1-2004/037150
- WO-A2-2006/091243
- US-A- 2 134 646
- US-A- 3 368 556
- US-A- 3 744 491
- US-A- 4 227 524
- US-A- 4 509 513
- US-A- 5 060 644
- US-A- 5 458 562
- US-A1- 2006 185 670

## Description

### BACKGROUND OF THE INVENTION

Hyperbaric chambers are device which create sealed environments for the application of therapeutic gases to hasten healing of lesions or wounds on a patient's body. As described in U.S. Patent No. 5,060,644, entitled, "Hyperbaric Chamber Apparatus," the disclosure of which is incorporated herein by reference, the introduction of pressurized gas, such as oxygen into such an encapsulated environment promotes healing of various types of lesions and wounds.

When hyperbaric chambers were first introduced for healing of lesions or wounds, they encompassed the entire body. As time progressed, hyperbaric chambers became more sophisticated, and topical hyperbaric chambers were developed, as described in U.S. Pat. No. 5,154,697 entitled, "Collapsible Topical Hyperbaric Apparatus" and 4,801,291, entitled, "Portable Topical Hyperbaric Apparatus," which are incorporated by reference herein.

The current re-usable wound treatment devices typically have rigid transparent panels affixed to a rigid frame. Seals placed between the panels and the frames have a tendency to leak with repeated use as the seals deform over time. Also, the panels have a tendency to loosen their seal due to the cyclical pressures in the device. Therefore, a re-usable wound treatment device is desired that can effectively and efficiently withstand the operational pressures, as well as provide an effective hermetic seal, even as the seals deform with use.

Further, some re-usable wound treatment devices form seals about the limb of a patient using tape. Often, these tape seals leak and come undone, allowing valuable treatment gas to escape. Accordingly, a seal is desired that can effectively seal the device against the limb without the use of tape.

US 5,458,562 describes a circulation enhancing device in the form of an airtight boot contoured to the injured foot, a pulsed synchronized tourniquet for inhibiting blood flow to the injured foot during an over-pressure cycle and a controlled circuit which monitors the heart's systolic and diastolic pressure pulsations and provides electrical control signals to the pressure modulator to assure that the over-pressure and vacuum pulses are cyclic and in synchronism with the heart's systolic and diastolic pressure pulsations. The circulation enhancing boot comprises a pair of boot halves joined together by a hinge assembly located at the back of the boot. The boot has a pneumatic cuff attached to the inner surface at a top portion of the boot, which cuff may be inflated so that the cuff encircles the leg forming an airtight seal about the leg.

WO 2006/091243 describes a hyperbaric oxygen device comprising an enclosure including a collapsible bag defined by two sheets of fluid-impervious material sealed together at both ends such that gas can be delivered between the sheets to inflate the enclosure to a rigid state and maintain the enclosure in the rigid state when oxygen pressure in the interior of the enclosure is cycled between ambient pressure and above ambient pressure.

### SUMMARY OF THE INVENTION

The present invention is set out in the appended claims. Described herein is a wound treatment device which includes a rigid housing having a housing wall with a first end and a second end forming a chamber therebetween. The device can further include an inflatable cuff releasably coupled to the housing wall at the first end. The housing wall can include a sidewall having an opening, a cover for closing the opening, and a cam mechanism for releasably fastening the cover to the sidewall surrounding the opening. The cam mechanism can include a cam having a flat surface, a swivel rod disposed within the cam for coupling with the housing, and a handle for rotating the cam.

Described herein is a wound treatment device which can include a rigid housing having a housing wall with a first end and a second end forming a chamber therebetween, an inflatable cuff releasably coupled to the housing wall at the first end, and an end cap coupled to the housing wall at the second end having a semispherical body extending out of the chamber.

Described herein is a rigid wound treatment device which can include a cylindrical wall having spaced apart ends forming a chamber therebetween, and an inflatable cuff releasably coupled to one of the ends and extending in the chamber.

### BRIEF DESCRIPTION OF THE DRAWINGS

The various objects, advantages and features of this invention will be more fully apparent from a reading of the following detailed description in conjunction with the accompanying drawings in which like reference numerals refer to like parts, and in which:
FIG. 1 is schematic illustration of a patient receiving wound treatment using a wound treatment device according to an embodiment of the present invention.
FIG. 2 is a perspective view of a housing for the wound treatment device of FIG. 1.
FIG. 3 is an exploded perspective view of the housing of FIG. 2.
FIG. 4 is a perspective view of a cuff seal assembly.
FIG. 5 is a side elevational view of the cuff seal assembly in an unassembled state.
FIG. 6 is a side elevational view of the cuff seal assembly in an assembled state.
FIG. 7 is an elevational view of an access port and cover of the device of FIG. 1, according to an embodiment of the present invention.
FIG. 8 is an elevational view of the cover of FIG. 7.
FIG. 9 is an elevational view of the access port of FIG. 7.
FIG. 10 is a perspective exploded view of a clamp, in accordance with an embodiment of the present invention.
FIGS. 11A, 11B, 11C and 11D are enlarged side elevational views of the clamp of FIG. 10, shown sealing the access port with the cover.
FIG. 12 is a perspective view of another embodiment of the present invention.

### DETAILED DESCRIPTION

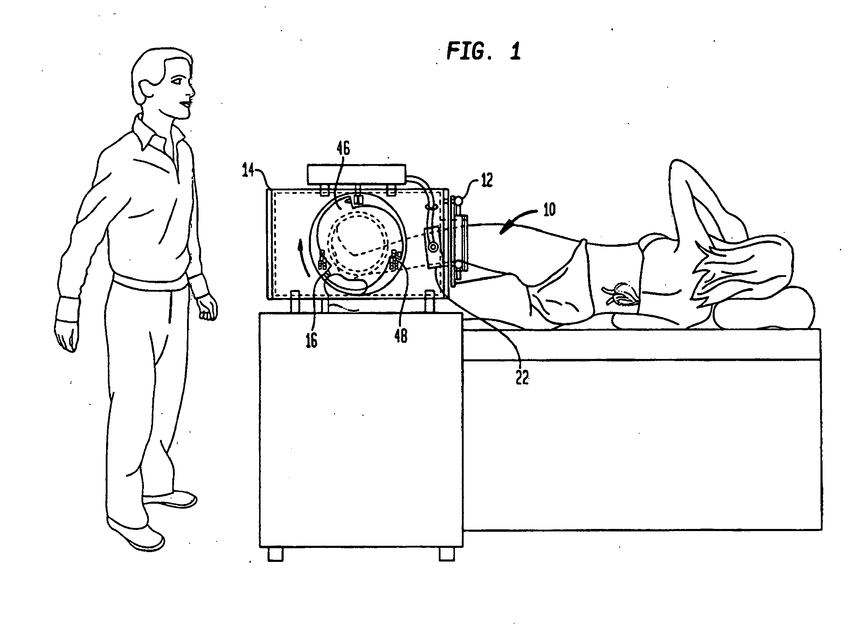

FIG. 1 illustrates a patient receiving hyperbaric therapy on a limb such as the patient's leg. The limb is placed inside a hyperbaric wound treatment device 10 constructed according to an embodiment of the present invention. Although embodiments of the invention disclosed herein describe hyperbaric treatment, any type of wound treatment may be used such as compression therapy or negative pressure therapy and the like.

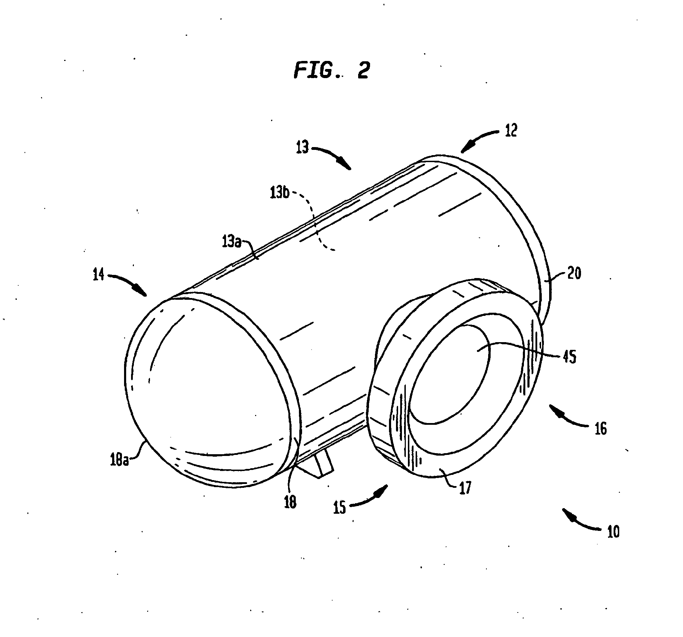

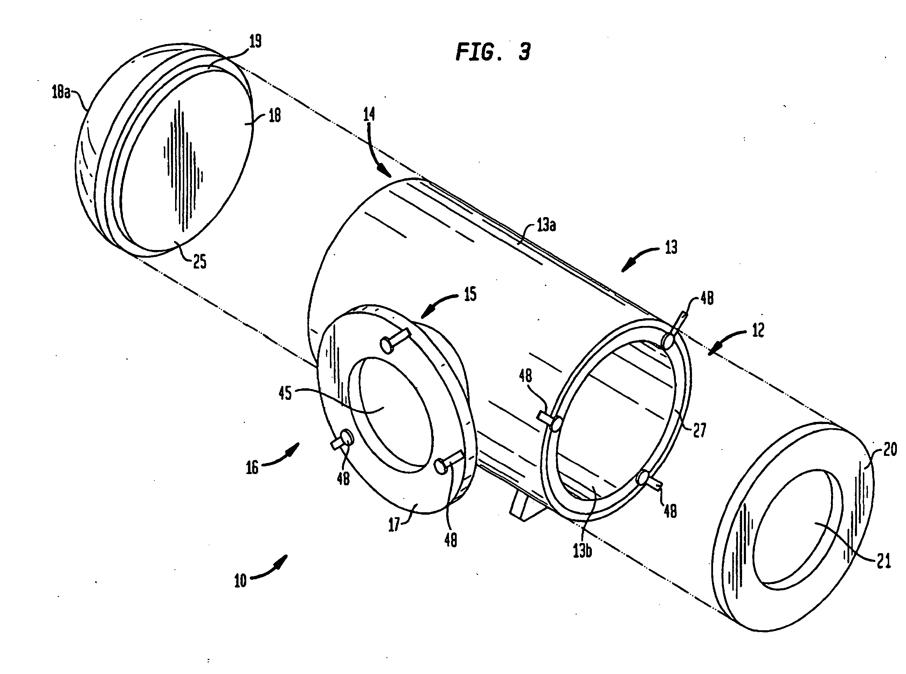

Referring more particularly to FIGS. 2 and 3, device 10 includes an elongated housing 13 having open ends 12, 14. Housing 13 is formed from a generally continuous housing wall 13a that defines an internal chamber 13b having a generally cylindrical shape for evenly distributing the pressures that are employed during hyperbaric therapy. Housing wall 13a can be formed from a rigid polymeric material, such as cast acrylics or a like material capable of withstanding the pressures employed during treatment. Further, wall 13a can be transparent to allow visual access into chamber 13b. Optionally, housing 13 can include a side access port 16 through which a clinician can have access to the wound on the patient's limb.

Mounted to one open end of housing 13 is a first end cap 18 that closes and seals the open end 14 of the housing. The first end cap 18 has a circular perimeter that generally matches the outer diameter of the cylindrical housing wall 13a and a lip 19 forming a cylindrical protrusion 25 that extends slightly into the open end 14 of the housing 13. The first end cap 18 can be releasably or fixedly secured to the housing by any suitable means and may include a gasket (not shown) located, about the lip 19. Permanent attachment may be achieved by gluing or heat welding, while releasable attachment may include the use of clamps (not shown) and the like. Further, the first end cap 18 can include a semi-spherical shaped body 18a that projects outwardly from the end cap. The semi-spherical shape of the body 18a is advantageous for withstanding the pressures within housing 13. Optionally, the thickness at the center of the end cap 18 can be greater than the thickness of the housing wall 13a. This greater thickness is advantages for allowing the first end cap 18 to withstand the pressures within the housing 13. In the illustrated embodiment, a second end cap 20 is similarly circular with an opening 21, optionally being a central opening sized to receive an appendage of a patient.

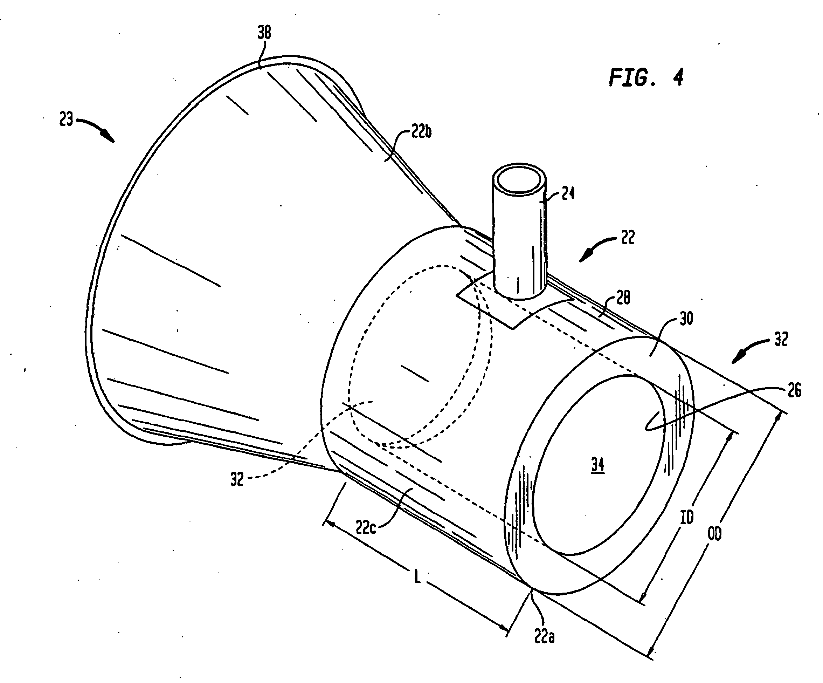

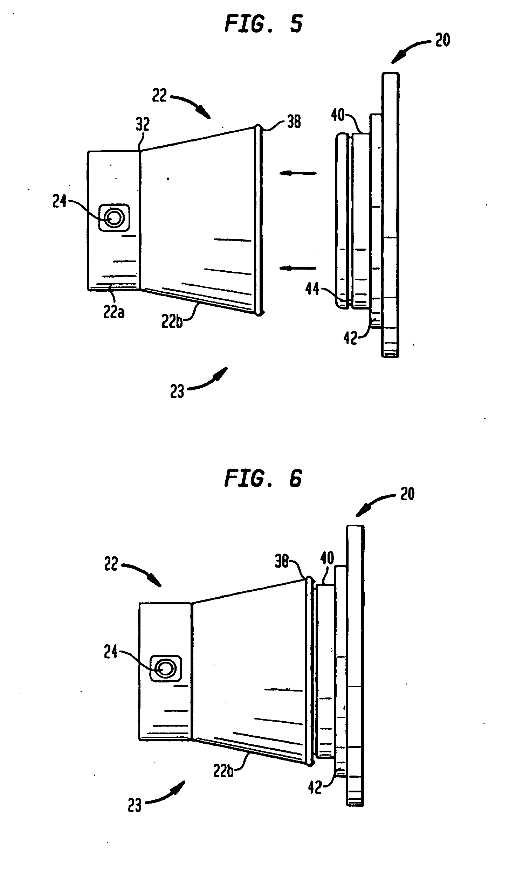

As best seen in FIGS. 5 and 6, the second end cap 20 attaches to a cuff seal assembly 23 to seal against the appendage. The cuff seal assembly 23 includes a cuff 22 removably attached to the second end cap 20 by a connecting member 22b. Cuff 22 and connecting member 22b are located and mounted inside chamber 13b by means of the second end cap 20. Cuff 22 is formed from a generally cylindrical shaped tubular member 22a, as shown in FIG. 4, having inner and outer concentric tubular walls 26 and 28. The cuff 22 has a first open end 32 for receiving a limb in passageway 34 formed by the tubular member 22a. The tubular member 22a is coupled to the second end cap 20 by a connecting member 22b in the form of a frusto-conical sleeve.

The inner and outer tubular walls 26 and 28 of the cuff 22 are sealed together to form an inflatable chamber 22c therebetween as shown in FIG. 4. To inflate cuff 22, outer tubular wall 28 includes a valve 24 which is in fluid communication with chamber 22c for inflating the cuff 22 with a gas, such as oxygen or air. Upon inflation, inner wall 26 of the cuff 22 expands inwardly to form a seal, such as a hermetic seal depending upon the inflation pressure, against the limb disposed in passageway 34 of the cuff 22.

Inner and outer tubular walls 26 and 28 may be joined and sealed together at their respective ends directly or indirectly by an interconnecting sidewall 30 to form chamber 22c, which can be inflated as described below. The cuff is preferably made from synthetic thermoplastic material which can be RF welded, thermal bonded or adhesive bonded. The cuff 22 has a length L, an inside diameter (ID) and an outside diameter (OD). The inside diameter (ID) is formed from the inside tubular wall 26 and the outside diameter (OD) is formed from the outside tubular wall 28.

As noted above, cuff 22 is inflated using valve 24, which opens and closes fluid communication between a pressurized fluid supply (not shown) and chamber 22c. Air or any suitable gas is introduced into chamber 22c through valve 24 between the inside and outside tubular walls 26 and 28 to thereby inflate the cuff 22. Prior to inflation, the inside diameter (ID) of the inner tubular wall is X. This ensures that the cuff 22 inner diameter is large enough to accommodate sliding a limb through the cuff 22, and hence reduce the trauma to the patient's appendage. Upon inflation, the inner tubular wall 26 is compressed such that its inside diameter reduces to an inside diameter less than X. The inside diameter then decreases when inflated enough to snugly encircle and seal against the appendage or limb being treated, once the limb has been inserted.

In a further embodiment, cuff 22 is formed from a flexible elastic material, such as a rubber or latex material, with an outside tubular wall 28 and the side wall 30 having a thickness greater than the thickness of the inside tubular wall 26. This increase in thickness results in greater stiffness in walls 28 and 30 such that when the cuff 22 is inflated, walls 28 and 30 resist flexure and generally maintain their size and dimension. This results in inner tubular wall 26 expanding toward the passageway 34 to accommodate the inflation pressure and, thus decreasing the inner diameter. Due to its reduced thickness, the inside tubular wall 26 will stretch where needed, to accommodate the surface topology of the limb or appendage while still sealing against the limb or appendage. By providing a relatively flexible inner tubular member, cuff 22 can seal against any variations in limb size or shape, such as a knee or ankle, along the length L of the cuff 22.

To couple the open end 32 of the cuff 22 to the second end cap 20, the frusto-conical connecting member 22b extending from the cuff is provided with a lip 38 formed around its distal edge. The frusto-conical connecting member 22b is similarly formed from a flexible elastic material, such as a rubber or latex material. The member 22 may be integrally formed with a portion of the tubular member 22a, or joined thereto by any suitable means, such as RF welding, thermal bonding or glueing and the like. Lip 38 may be formed as an enlarged edge, such as a rolled edge of the frusto-conical member 22b, or as a thickened edge portion of the frusto-conical member. As understood from FIGS. 5 and 6, lip 38 releasably engages the second end cap 20. To this end, the second end cap 20 may have an inwardly projecting annular skirt 40 that inserts into opening 21 of end cap 20 or which is formed therewith. Further, end cap 20 includes a gasket 42 for sealing, such as hermetically sealing, against the first end 12 of the housing 13 when end cap 20 is mounted to the housing.

The skirt 40 includes an annular groove 44 that receives and is releasably engaged by lip 38. Thus, when lip 38 of the frusto-conical member 226 is pulled over skirt 40 of end cap 20, lip 38 is received into groove 44, and the frusto-conical member 22b wraps around the entire circumference of the groove 44 and annular skirt 40. Further, the inner diameter of the frusto-conical member 22b may be smaller than the outside diameter of skirt 40 so that the frusto-conical member must be stretched to fit over the skirt 40 and thereby provide a compression fit with skirt 40.

Typically, cuff 22 is mounted to the second end cap 20 before the second end cap is mounted to the open end 12 of the housing 13. Thus, when cuff 22 is mounted on the second end cap 20 and inserted into housing 13, the cuff 20 is received entirely within the internal chamber 13b. However, the orientation of the cuff 22 and second end cap 20 can be reversed such that the cuff can be wholly external to the housing 13 and still create a hermetic seal against the limb. Further, the cuff 22 can be configured to be only partially received within the housing 13.

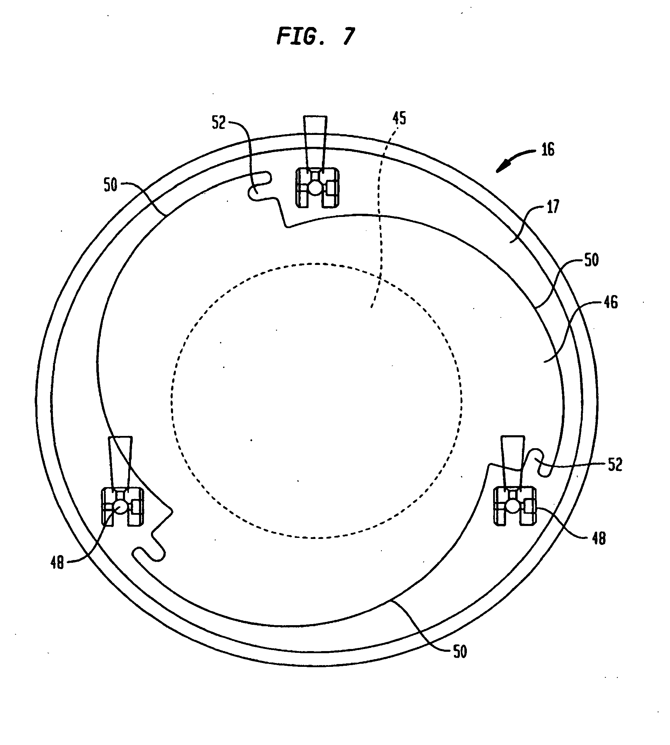

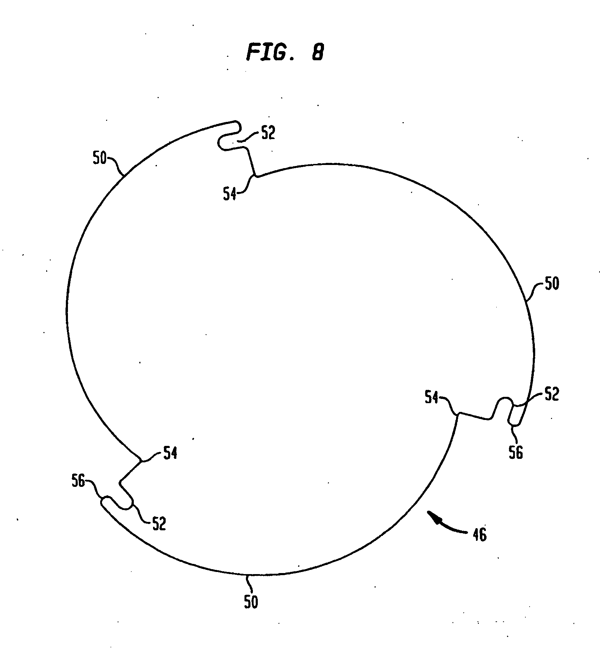

As noted above, housing 13 optionally includes one or more access ports 16. As best seen in FIGS. 2 and 3, an extension 15 can be formed on a side of the housing 13 to forms the access port 16. Access port 16 includes an opening 45 in housing wall 13a and a removable cover 46 (See FIGS. 7 and 8). Optionally, access port 16 is large enough to permit a clinician sufficient access to the appendage and, further, to the wound to provide medication for the wound or to change dressings. Cover 46 may be secured over opening 45 to close the access port 16 using a plurality of clamps 48, as shown in FIG. 7. A lip 17 (FIGS. 2 and 3) can be formed at the access port 16 to accommodate the cover 46. Thus, the cover 46 may be releasably coupled to the lip 17 using clamps as described below.

Cover 46 may be formed from a suitable material, such as a polymer, including cast acrylic similar to housing wall 13a. Additionally, cover 46 may be transparent so as to provide visual access to the wound within housing 13. Although shown having a circular shape, the access port 16 can have any shape as desired such as square, oval, polygonal and the like.

There may be a flexible, polymer gasket or a seal between the cover 46 and the lip 17 to provide a seal, such as a hermetic seal, around opening 45. This gasket prevents or reduces treatment gas from escaping. Due to their inherent nature, gaskets can deform over time, permitting valuable treatment gas to escape. Therefore, in an embodiment of the present invention, clamps 48 are constructed in a manner that can compensate for the natural deformity in the gaskets.

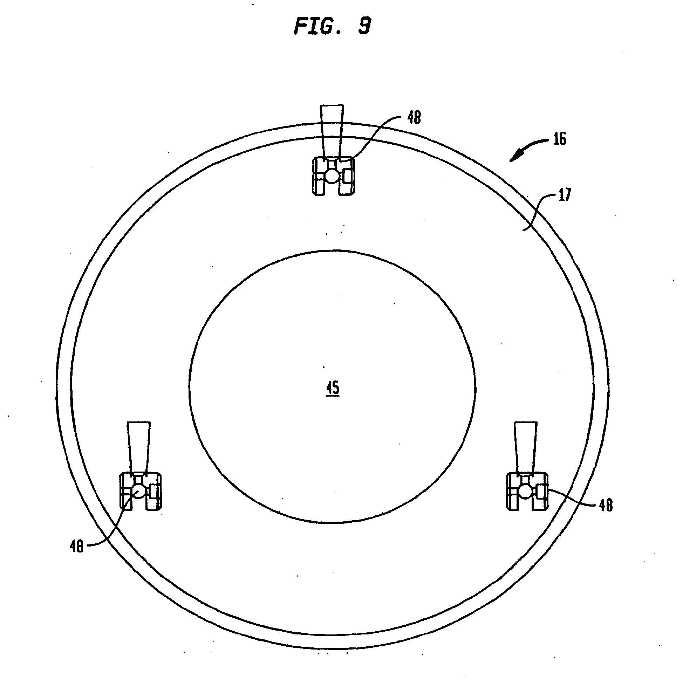

Referring to FIGS. 7-9, cover 46 has a plurality of arcuate sides 50 each with a corresponding plurality of slots 52. Although three sides 50 and three slots 52 are illustrated herein, any numbers of sides and corresponding slots can be utilized. Each side 50 has a first diameter near an inside edge 54 of the respective side 50 and a second larger diameter near an outside edge 56 adjacent the slot 52 such that the sides flare radially outwardly with increasing diameter between the first and second diameters.

The slots 52 are configured to mate with clamps 48 which are disposed on the lip 17. The number of slots 52 correspond to the number of clamps 48. It should be understood that the number of slots 52 or clamps 48 can be varied correspondingly, The cover 46 is placed over the opening 45 against the lip 17 and turned in a clockwise manner so that slots 52 of the cover mate with the clamps 48. The cover 46 can be easily configured so that turning it counter clockwise will be equally effective. Then the clamps 48 fasten the cover 46 to the lip 17, to be described more fully below. As stated previously and not evident from FIGS. 7-9, a gasket is placed between the cover 46 and the lip 17.

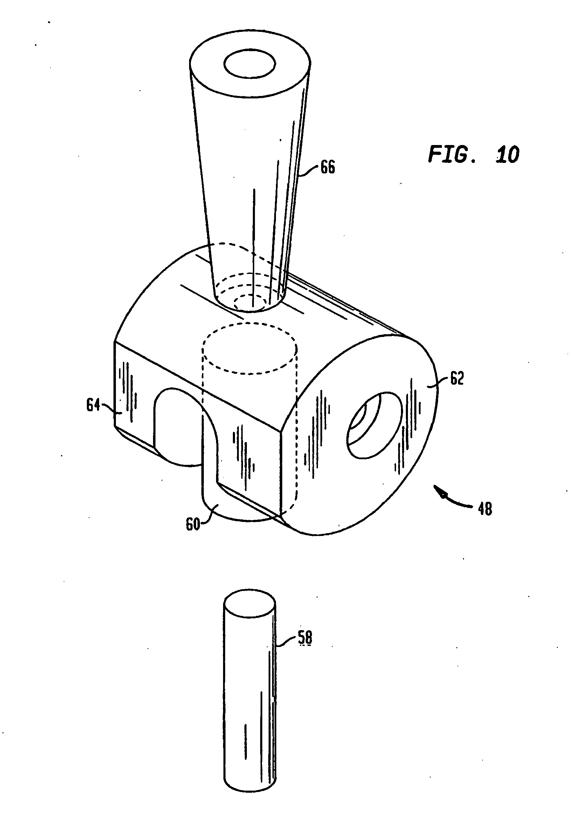

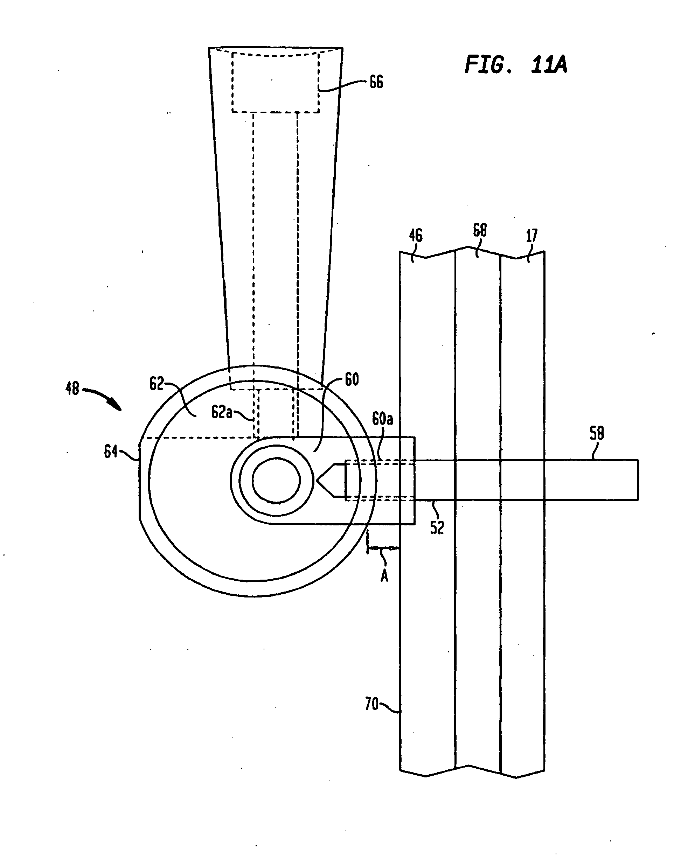

FIGS. 10 and 11A illustrate details of the clamp mechanism where the clamp 48 includes a mounting stud 58, a swivel rod 60, which is internally threaded, an eccentric cam 62 having a flat surface 64, and a handle 66. Each mounting stud 58 is fastened at one end to the lip 17 of the access port 16. The other end of the stud 58 is threadably engaged within a threaded socket 60a formed in the swivel rod 60. similarly, handle 66 threads into a threaded socket 62a in cam 62.

As seen in FIGS. 11A-11D, in a side view of the access port 16 and the cover 46, slots 52 of cover 46 are arranged so that they align with the mounting studs 58. Cover 46 is positioned in the space created around the mounting stud 58 between cam 62 and the lip 17. Positioned between the lip 17 and the cover 46 is a gasket 68. Optionally, gasket 68 includes a hole (not shown) for fitting over the mounting stud 58. Generally, the cover 46 is placed over the access port 16, the cover's slots 52 mate with the mounting stud 58 of the clamp 48 and the handle 66 is rotated to close the clamp 48 and fasten the cover 46 to the lip 17.

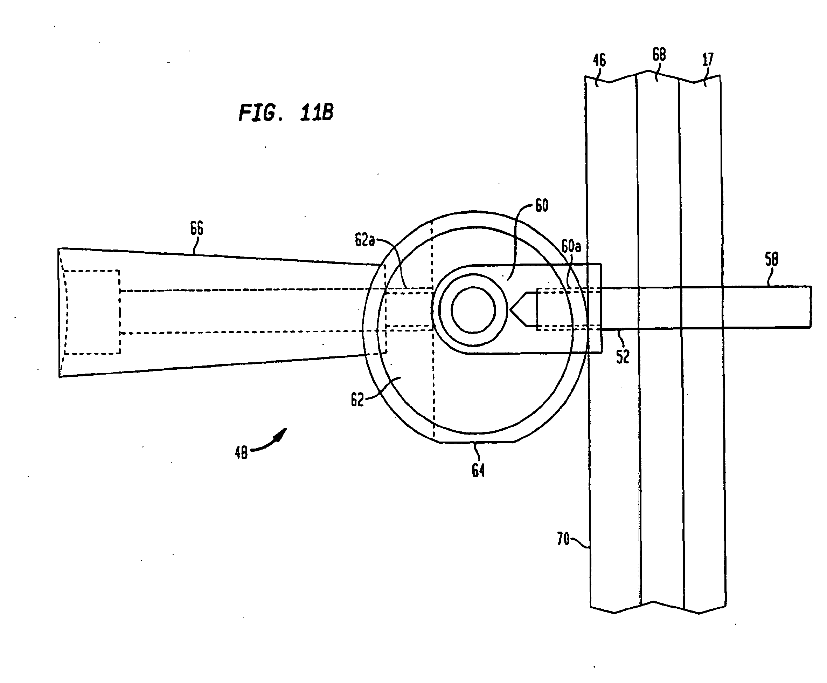

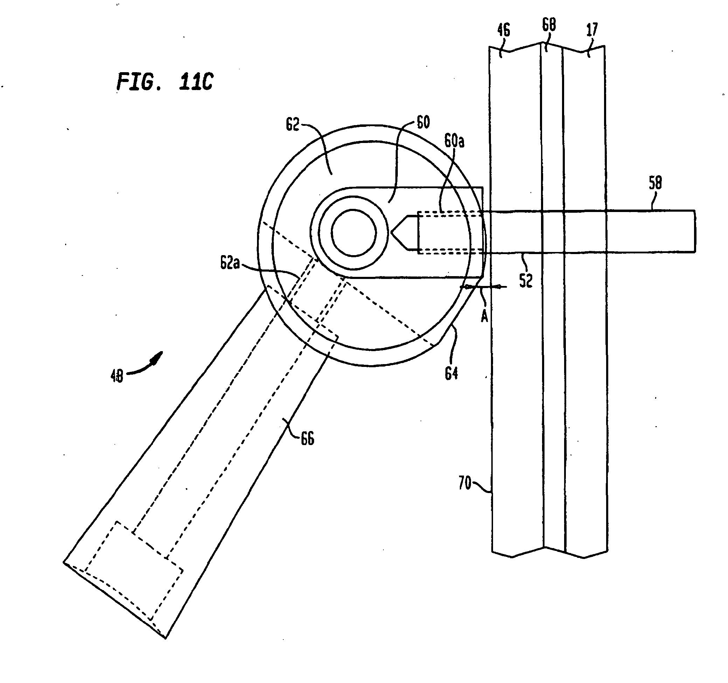

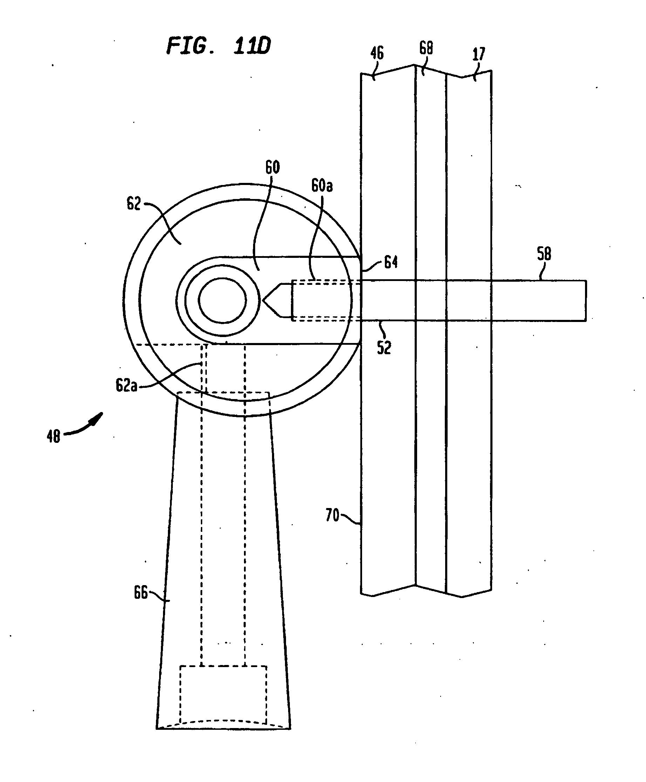

FIGS. 11A, 11B, 11C and 11D illustrate different positions when the handle 66 rotates the cam 62 around the mounting stud 58. Referring to FIG. 11A, handle 66 is at a first open, unclamped position, wherein the handle 66 is at a 90° angle from the mounting stud 58. In this position a wide gap A is formed between cam 62 and an outer surface 70 of the cover 46. The gap compensates for changes in the thickness of the gasket 68.

Upon rotation of handle 66 to a second, mid-way position, depicted in FIG. 11B, the handle is at a 180° angle from the mounting stud 58. In this position, the gap A is reduced and the eccentric cam 62 has begun compressing the gasket 68. This cam action is achieved in part by the shape of cam 62 but also by the offset of pivot axis 62a of pivot axis cam 62.

In a third position, depicted in FIG. 11C, the handle 66 is at an angle between 180° and 270° of the mounting stud 58. The cam 62 has compressed the gasket 68 to an even greater extent reducing gap A even further. Finally, as depicted in FIG. 11D, the handle 66 is rotated to be at an angle of 270° from the mounting stud 58. In this position, the flat surface 64 is parallel to and faces the gasket 68. At this last and locked position, cam 62 exerts the greatest compression force against gasket 68; gap is at or near zero. Thus, at various positions, the cam 62 accommodates changes in the thickness of the gasket 68, reducing the size of gap A.

The flat surface 64 can be machined or cast on an outside portion of the cam 62 where the flat surface 64 provides the maximum sealing pressure. This flat surface 64 prevents the cam 62 from working its way loose. Thus, this flat surface 64 effectively locks the cam 62 into position.

While cam clamp 48 is shown being used with a rigid hyperbaric wound treatment device 10, it should be noted that this cam clamp 48 can be used in any application requiring seals that change in thickness over time. Preferably, the clamp 48 is formed of a metal that is resistant to rust and moisture. For example, 300 series stainless steel is preferred.

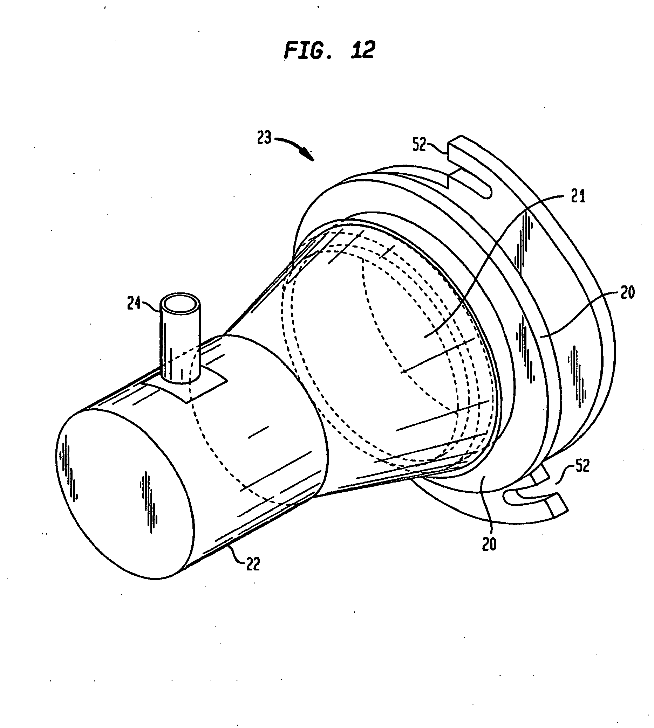

Although the clamps 48 have been discussed with respect to the closure of the access port 16 with the cover 46, a similar configuration can be used at the second end 12 of the housing to attach the second end cap 20 to the open end 12, as depicted in FIG. 12. In this instance, the open end 12 can include a lip 27 similar to lip 17 and have clamps 48 mounted thereon. The second end cap 20 can be configured in a manner similar to that of cover 46. Generally, the second end cap 20 can have arcuate sides 50 with slots 52 for mating with the clamps 48 disposed on the second end lip 27. The primary difference between the second end cap 20, in this embodiment and the cover 46 discussed earlier is that the second end cap 20 is open at the center 21 to accommodate the limb. A gasket (not shown) can be placed between the lip 27 and the second end cap 20. A hermetic seal between the housing and the second end cap 20 can be formed by the cuff 22 in the manner discussed previously.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A chamber suitable to create a sealed environment for the application of therapeutic gases, comprising:
a rigid housing (13) having spaced apart ends (12, 14) forming a chamber therebetween;
an end cap (20) coupled to one end (12) of the housing, wherein the end cap has an opening (21), the end cap (20) being releasably coupled to the rigid housing (13); and
an inflatable cuff (22) releasably coupled to the end cap (20) for sealing against a limb.

2. The device of claim 1, wherein the housing (13) further includes an access port (16) on a side of the housing, and an access port cover (46) releasably coupled to the access port (16), wherein the access port is configured with a clamp (48) for releasably attaching the access port cover (46) thereto.

3. The device of claim 1, further including a flexible sleeve (22b) having a first portion that releaseably couples to the end cap (20) and a second portion that couples to the inflatable cuff (22).

4. The device of claim 3, wherein the flexible sleeve has a frusto-conical shape.

5. The device of claim 3, wherein the end cap (20) includes at least one arcuate side (50) having a first end having a first radius from a center of the end cap (20), and a second end having a second radius from the center, wherein the first radius is smaller than the second radius, wherein the end cap (20) includes a slot (52) near the second end of the arcuate side (50).

6. The device of claim 3, wherein the first portion has a flexible lip (38) that engages a groove (44) disposed on an inside surface of the end cap (20).

7. The device of claim 1, wherein the inflatable cuff includes an outer wall (28) coupled to an inner wall (26) forming a cuff interior therebetween adaptable for fluid communication with an inflating gas source.

8. The device of claim 7, further including a sidewall (30) attached between said outer wall (28) and said inner wall (26).

9. The device of claim 8, wherein the outer wall (28) and sidewall (30) are more rigid than the inner wall (26).

10. The device of claim 1, further including an end cap (18) comprising a semispherical body (18a) coupled to one (14) of the spaced apart ends.

11. The device of claim 1, further including a cam mechanism for releasably fastening the end cap to one end of the housing.

12. The device of claim 11, wherein the cam mechanism comprises:
a cam (62) having a flat surface (64);
a swivel rod (60) disposed within the cam (62) for coupling with the housing; and
a handle (66) for rotating the cam.

13. The device of claim 1, wherein the inflatable cuff is arranged for use either partially or wholly inside the chamber.

14. The device of claim 1, wherein said housing (13) is cylindrical.

## Patentansprüche

1. Kammer, die zum Erzeugen einer abgedichteten Umgebung zum Applizieren von therapeutischen Gasen geeignet ist und Folgendes umfasst:
ein starres Gehäuse (13) mit beabstandeten Enden (12, 14), die eine Kammer dazwischen bilden;
eine Endkappe (20), die mit einem Ende (12) des Gehäuses gekoppelt ist, wobei die Endkappe eine Öffnung (21) hat, wobei die Endkappe (20) lösbar mit dem starren Gehäuse (13) gekoppelt ist; und
eine aufblasbare Manschette (22), die lösbar mit der Endkappe (20) gekoppelt ist, um gegen eine Gliedmaße abzudichten.

2. Vorrichtung nach Anspruch 1, wobei das Gehäuse (13) ferner eine Zugangsöffnung (16) auf einer Seite des Gehäuses und eine lösbar mit der Zugangsöffnung (16) gekoppelte Zugangsöffnungsabdeckung (46) aufweist, wobei die Zugangsöffnung mit einer Klammer (48) zum lösbaren Anbringen der Zugangsöffnungsabdeckung (46) daran konfiguriert ist.

3. Vorrichtung nach Anspruch 1, die ferner eine flexible Hülse (22b) mit einem lösbar mit der Endkappe (20) koppelbaren ersten Abschnitt und einem mit der aufblasbaren Manschette (22) koppelbaren zweiten Abschnitt aufweist.

4. Vorrichtung nach Anspruch 3, wobei die flexible Hülse eine Kegelstumpfform hat.

5. Vorrichtung nach Anspruch 3, wobei die Endkappe (20) wenigstens eine bogenförmige Seite (50), die ein erstes Ende mit einem ersten Radius von einer Mitte der Endkappe (20) hat, und eine zweite Seite mit einem zweiten Radius von der Mitte aufweist, wobei der erste Radius kleiner ist als der zweite Radius, wobei die Endkappe (20) einen Schlitz (52) in der Nähe des zweiten Endes der bogenförmigen Seite (50) aufweist.

6. Vorrichtung nach Anspruch 3, wobei der erste Abschnitt eine flexible Lippe (38) aufweist, die in eine auf einer Innenfläche der Endkappe (20) angeordnete Nut (44) eingreift.

7. Vorrichtung nach Anspruch 1, wobei die aufblasbare Manschette eine Außenwand (28) aufweist, die mit einer Innenwand (26) gekoppelt ist, die ein Manschetteninneres dazwischen bildet, das für eine Fluidverbindung mit einer Aufblasgasquelle adaptierbar ist.

8. Vorrichtung nach Anspruch 7, die ferner eine Seitenwand (30) aufweist, die zwischen der genannten Außenwand (28) und der genannten Innenwand (26) angebracht ist.

9. Vorrichtung nach Anspruch 8, wobei die Außenwand (28) und die Seitenwand (30) starrer sind als die Innenwand (26).

10. Vorrichtung nach Anspruch 1, die ferner eine Endkappe (18) aufweist, die einen halbkugelförmigen Körper (18a) umfasst, der mit einem (14) der beabstandeten Enden gekoppelt ist.

11. Vorrichtung nach Anspruch 1, die ferner einen Nockenmechanismus zum lösbaren Befestigen der Endkappe an einem Ende des Gehäuses aufweist.

12. Vorrichtung nach Anspruch 11, wobei der Nockenmechanismus Folgendes umfasst:
eine Nocke (62) mit einer flachen Oberfläche (64);
einen Drehstab (60), der zum Koppeln mit dem Gehäuse in der Nocke (62) angeordnet ist; und
einen Griff (66) zum Drehen der Nocke.

13. Vorrichtung nach Anspruch 1, wobei die aufblasbare Manschette zur Verwendung entweder teilweise oder gänzlich innerhalb der Kammer angeordnet ist.

14. Vorrichtung nach Anspruch 1, wobei das genannte Gehäuse (13) zylindrisch ist.

## Revendications

1. Chambre adaptée pour créer un milieu ambiant étanche en vue de l'application de gaz thérapeutiques, comprenant :
un logement rigide (13) possédant des extrémités espacées l'une de l'autre (12, 14) lesquelles forment une chambre entre elles ;
un capuchon d'extrémité (20) couplé à une extrémité (12) du logement, alors que le capuchon d'extrémité présente une ouverture (21), le capuchon d'extrémité (20) étant couplé de façon détachable au logement rigide (13) ; et
une manchette gonflable (22) couplée de façon détachable au capuchon d'extrémité (20) pour se sceller contre un membre du corps humain.

2. Dispositif selon la revendication 1, le logement (13) incluant en outre un port d'accès (16) sur un côté du logement, et un couvercle de port d'accès (46) couplé de façon détachable au port d'accès (16), alors que le port d'accès est configuré pour comporter une pince (48) permettant d'y attacher de façon détachable le couvercle de port d'accès (46).

3. Dispositif selon la revendication 1, incluant en outre un manchon souple (22b) lequel possède une première portion qui s'accouple de façon détachable au capuchon d'extrémité (20), et une deuxième portion qui s'accouple à la manchette gonflable (22).

4. Dispositif selon la revendication 3, le manchon souple ayant une forme frusto-conique.

5. Dispositif selon la revendication 3, le capuchon d'extrémité (20) incluant au moins un côté arqué (50) lequel possède une première extrémité qui a un premier rayon par rapport à un centre du capuchon d'extrémité (20), et une deuxième extrémité qui a un deuxième rayon par rapport au centre, alors que le premier rayon est plus petit que le deuxième rayon, le capuchon d'extrémité (20) incluant une fente (52) à proximité de la deuxième extrémité du côté arqué (50).

6. Dispositif selon la revendication 3, la première portion possédant une lèvre souple (38) qui s'emboîte avec une rainure (44) disposée sur une surface interne du capuchon d'extrémité (20).

7. Dispositif selon la revendication 1, la manchette gonflable incluant une paroi externe (28) couplée à une paroi interne (26) lesquelles forment entre elles une face intérieure de manchette apte à être adaptée pour une communication fluidique avec une source de gaz de gonflage.

8. Dispositif selon la revendication 7, incluant en outre une paroi latérale (30) laquelle est attachée entre ladite paroi externe (28) et ladite paroi interne (26).

9. Dispositif selon la revendication 8, la paroi externe (28) et la paroi latérale (30) étant plus rigides que la paroi interne (26).

10. Dispositif selon la revendication 1, incluant en outre un capuchon d'extrémité (18) lequel comporte un corps demi-sphérique (18a) qui est couplé à l'une (14) des extrémités espacées l'une de l'autre.

11. Dispositif selon la revendication 1, incluant en outre un mécanisme à came pour assujettir de façon détachable le capuchon d'extrémité à une extrémité du logement.

12. Dispositif selon la revendication 11, le mécanisme à came comprenant :
une came (62) possédant une surface plate (64) ;
une tige pivotante (60) disposée à l'intérieur de la came (62) en vue d'un accouplement avec le logement ; et
une poignée (66) pour faire tourner la came.

13. Dispositif selon la revendication 1, la manchette gonflable étant agencée en vue d'une utilisation, soit partiellement soit intégralement, à l'intérieur de la chambre.

14. Dispositif selon la revendication 1, ledit logement (13) étant cylindrique.
